# EUROPÄISCHE PATENTSCHRIFT

(11) **EP 2 831 563 B1**
(45) Veröffentlichungstag und Bekanntmachung des Hinweises auf die Patenterteilung: **25.03.2020**
(21) Anmeldenummer: 13722665.0
(22) Anmeldetag: 25.03.2013
(51) Int. Cl.: G01N 5/00, G01N 15/06, G01N 33/00

(54) **MESSVORRICHTUNG**
MEASURING DEVICE
DISPOSITIF DE MESURE

(30) Priorität: 27.03.2012 DE 102012006052
(43) Veröffentlichungstag der Anmeldung: 04.02.2015
(73) Patentinhaber: Wöhler Technik GmbH, 33181 Bad Wünnenberg (DE)
(72) Erfinder: ESTER, Stephan, 33181 Bad Wünnenberg (DE)
(74) Vertreter: Rieke, Andreas
(86) Internationale Anmeldenummer: PCT/DE2013/000163
(87) Internationale Veröffentlichungsnummer: WO 2013/143523

(56) Entgegenhaltungen:
- DE-A1- 19 727 969
- DE-A1-102006 026 002
- US-A- 4 442 699
- US-A- 6 016 688
- US-B1- 7 111 496
- US-B2- 7 947 503

## Beschreibung

Die Erfindung betrifft eine Messvorrichtung für die Staubmessung im Abgas von Kleinfeuerungsanlagen für feste Brennstoffe, aufweisend eine Messsonde und einen die Messsonde mit einer Wägevorrichtung mit einer Filtervorrichtung verbindenden, beheizten Ansaugschlauch.
Im Jahre 2010 erfolgte die Novellierung der ersten Verordnung zur Durchführung des Bundes-Immissionsschutzgesetzes, die Verordnung über kleine und mittlere Feuerungsanlagen, 1.BImSchV, mit der ein wesentlicher Beitrag zur Reduzierung der Feinstaubimmissionen aus Kleinfeuerungsanlagen erreicht werden soll. So kann insbesondere der Einsatz von Holz in Feuerungsanlagen im Anwendungsbereich der Verordnung über kleine und mittlere Feuerungsanlagen einen wichtigen Beitrag zur Erreichung der Klimaschutzziele leisten. Allerdings kann die Verfeuerung von Biomasse durch die Freisetzung verschiedener Luftschadstoffe zu gesundheitsgefährdenden Immissionen wie bspw. auch Feinstaub führen.
Um die Klimaziele zu erreichen, verlangt die 1.BImSchV eine regelmäßige Überwachung der Feuerungsanlagen und schreibt, abhängig vom Typ der Feuerungsanlagen, Höchstwerte für den Feinstaubgehalt im Abgas vor.
Für neuere Feuerungsanlagen liegen diese Grenzwerte ab dem Jahr 2015 bei 0,02 g/m³. Diese Grenzwerte stellen für die Messtechnik durchaus eine Herausforderung dar, wenn auch das Grundprinzip einfach und unverändert ist, nämlich eine vorgegebene Menge des Abgases einer Feuerungsanlage durch einen Filter zu leiten und die Massendifferenz vor und nach dem Messvorgang festzustellen, um den Gehalt an Feinstaub in dem Abgas zu bestimmen.

Um derartig geringe Staubmengen vermessen zu können, werden in der technischen Entwicklung derzeit optische Systeme favorisiert, beispielsweise beschrieben in der DE 10 2006 039670 A1, der DE 10 2005 009582 A1 oder der DE 6 9627922 T2.

Als nachteilig bei optischen Systemen zur Bestimmung einer Staubmenge in einem Abgas ist grundsätzlich die unterschiedliche Beschaffenheit der Staubpartikel anzusehen und damit auch deren Reflektionsverhalten, so dass insbesondere bei der Vermessung geringster Staubmengen dies auf optischem Wege grundsätzlich als nicht unproblematisch erscheint.

Eine Alternative zu herkömmlichen Waagen zeigt die DE 2 553 638 C2 auf. Dort ist eine Vorrichtung erläutert, die ein elastisches Element in Form eines hohlen Rohres aufweist, das sich ausgehend von einem eingespannten ersten Ende hin zu einem zweiten, freien Ende verjüngt. Das freie Ende des Rohrs ist aufgeweitet und trägt eine plattformartige Unterlage für ein zu verwiegendes Gut. Aus der Änderung der Resonanzfrequenz des schwingfähigen Rohres in einem belasteten und in einem durch das zu verwiegende Gut belasteten Zustand kann auf die Masse des zu verwiegenden Guts geschlossen werden. Ein exaktes Wägen einer Probe erscheint mit dieser Vorrichtung möglich, jedoch ist diese bekannte Vorrichtung grundsätzlich für eine Abgasanalyse einer Feststofffeuerung ungeeignet.

Für die Messvorrichtung nach der Erfindung ist bevorzugt ein Messverfahren vorgesehen, wie es vergleichbar in der DE 10 2007 041 369 A1 erläutert wird. Für die Bestimmung der Staubmenge in dem Abgas einer Feststofffeuerung durchströmt dort eine vorbestimmte Menge des Abgases ein schwingungsfähiges Röhrchen, woraufhin sich auf einer Filtervorrichtung des Röhrchens eine Staubmenge ablagert. Das Röhrchen wird in eine Schwingung versetzt und es wird aus der Abweichung von der vorbestimmten Resonanz- und/oder einer Eigenfrequenz des Röhrchens durch eine Auswertevorrichtung die Staubmenge berechnet.
Neben der Wägevorrichtung sind die Schlauchanschlüsse zwischen einer in den Abgasstrom einzubringenden Messsonde und der eigentlichen Messvorrichtung von erheblicher Bedeutung. Aus der gattungsbildenden US 7,947,503 B2 ist eine Messvorrichtung mit einer der DE 2 553 638 C2 vergleichbaren Wägevorrichtung bekannt, bei der zwischen einer Gaseintrittsöffnung und der Wägevorrichtung ein beheizter Ansaugschlauch vorgesehen ist. Durch das Beheizen des Ansaugschlauchs wird verhindert, dass sich vor der Wägevorrichtung bereits in dem Schlauch Partikel absetzen und das Messergebnis verfälschen. Die Messgenauigkeit wird durch diese Maßnahme deutlich erhöht.
Die aus der US 7,947,503 B2 bekannte Messvorrichtung ist allerdings nicht geeignet, Feinstaubmessungen gemäß der 1.BImSchV vorzunehmen.

US 4 442 699 A offenbart eine Vorrichtung zur Messung des Staubgehaltes von Gasströmungen.
Vor diesem technischen Hintergrund macht die Erfindung es sich zur Aufgabe, eine praxisgerechte Messvorrichtung zur Verfügung zu stellen, mit der in einfacher Weise, jedoch sehr genau, insbesondere die Feinstaubmessungen gemäß 1.BImSchV durchgeführt werden können.
Gelöst wird diese technische Problematik bei einer Messvorrichtung für die Staubmessung im Abgas von Kleinfeuerungsanlagen für feste Brennstoffe durch die Merkmale des Anspruchs 1.

Die Messvorrichtung nach der Erfindung weist eine Vielzahl von Vorteilen auf.

Der beheizte Schlauch und die thermische Isolation der Wägevorrichtung stellen nach einer Aufheizphase sicher, dass es kaum zu einer Kondensation oder dergleichen Niederschläge auf dem Weg von der Sonde bis hin zu dem Ort der Abscheidung des Feinstaubs und dessen Verwägung kommt.

Insbesondere kann damit auch auf Abscheider jedweder Art vor dem Ort der Verwägung verzichtet werden oder auf das Zumischen von Frischluft.

Um diese Aufheizphase durch ein Abgas zu vermeiden, ist bei einer bevorzugten Ausführungsform der Messvorrichtung nach der Erfindung weiter vorgesehen, dass die Wägevorrichtung in einer beheizbaren Hülse in dem Wiegemodul angeordnet ist.

Während der Ansaugschlauch während einer Messung durch die Beheizung kontinuierlich auf einer Solltemperatur von ca. 75°C gehalten wird, bedarf es bei der die Wägevorrichtung umgebenden Hülse lediglich eines impulsartigen Aufheizens vor der Messung, die ca. eine Minute dauert. Haben sich im Ansaugschlauch und in der Wägevorrichtung die Temperaturen stabilisiert, kann die eigentliche Messung erfolgen, wobei dann auch sichergestellt ist, dass sich weder im Ansaugschlauch noch in der Wägevorrichtung bedeutende Niederschläge absetzen.

Neben einer Bedienerfreundlichkeit der Messvorrichtung nach der Erfindung steht naturgemäß ein exaktes Vermessen eines Abgases insbesondere einer Kleinfeuerungsanlage für Festbrennstoffe im Vordergrund. Diese Messungen sollen bei einer Abgastemperatur von ca. 70° erfolgen. Damit auch der Ansaugschlauch entsprechend konditioniert ist, hat es sich als zweckmäßig erwiesen, dass die Beheizung des Ansaugschlauches geregelt erfolgt. Als Ist-Temperaturgeber ist ein Schlauchtemperatursensor vorgesehen, angeordnet zwischen der Wägevorrichtung und dem Schlauch. Insbesondere ist der Schlauchtemperatursensor mit der Wägevorrichtung und einem Ende des Ansaugschlauch lösbar verbunden, womit zum einen eine Beschädigung des Sensors bei einem Biegen des Ansaugschlauchs, einem Herabfallen oder dergleichen ausgeschlossen und zum anderen eine einfache Reinigung des Schlauchtemperatursensors und des Abgasschlauchs ermöglicht ist.

Die Wägevorrichtung selbst weist in der Hülse ein schwingungsfähiges Röhrchen angeordnet auf, das gasaustrittsseitig frei endend von einer aufgesteckten Filterpatrone abgeschlossen ist. Die Bestimmung der Staubmenge erfolgt dann vorzugsweise vergleichbar dem aus der DE 10 2007 041 369 A1 bekannten Verfahren, wobei durch die aufgesteckte Filterpatrone ein rascher Austausch derselben sichergestellt ist, da für jede Messung eine unbenutzte Filterpatrone erforderlich ist.

Die Bedienerfreundlichkeit wird dadurch gewährleistet, dass vorgesehen ist, dass das Wiegemodul eigenständig ausgebildet und kombiniert mit einem Anzeige- und Bedienmodul an einem teleskopierbaren Stativ aus einem Koffer ausziehbar ist.

Da das notwendige Equipment für die Durchführungen der Messungen gemäß 1.BImSchV vergleichsweise umfangreich ist und bspw. die hierfür vorzusehenden Pumpen von erheblichem Gewicht sind, ist es von Vorteil, die Messvorrichtung vollständig nebst Equipment in einem Koffer unterzubringen, beispielsweise in einem Alurahmenkoffer von ausreichender Stabilität. Zweckmäßigerweise kann dieser mit Rollen oder dergleichen unterseitig noch versehen sein.

Für die Durchführung von Messungen wird in einfacher Weise das Wiegemodul mit ausgebildeter Wägevorrichtung, kombiniert mit dem Anzeige- und Bedienteil, nach Öffnen des Koffers an einem teleskopierbaren Stativ ausgezogen, bis das Anzeige- und Bedienmodul für das Bedienpersonal in einer bequemen Arbeitshöhe ist. Gleichzeitig wird damit auch der Ansaugschlauch aus dem Koffer ausgezogen, der das Wiegemodul mit Wägevorrichtung mit der Messsonde verbindet. Derartig aus dem Koffer ausgezogen ist auch die Messsonde problemlos zugänglich und kann gegebenenfalls auch ausgewechselt werden.

Dabei hat es sich weiter als zweckmäßig erwiesen, wenn innerhalb des Koffers eine Haltevorrichtung für den beheizten Ansaugschlauch vorgesehen ist.

In konstruktiver Ausgestaltung hat es sich dabei bewährt, wenn das Wiegemodul mit dem Anzeige- und Bedienmodul an dem Stativ abnehmbar angeschlossen ist und beispielsweise, bauseitig bedingt, an einem weiteren Stativ abgesetzt von dem Koffer verwendet wird. Hierzu, wie auch für das Ausziehen, ist das Wiegemodul mit einer Handhabe versehen. An dieser wird sich auch ein Bedienhebel für beispielsweise eine Klemmvorrichtung oder dergleichen befinden, um eine Arretierung an einem Stativ zu lösen.

Von dem Wiegemodul führt ein Verbindungsschlauch in das Kofferinnere zu der dort angeordneten Pumpe. Um diese Pumpe sowie dort weiter angeordnete Sensoren zu schützen, ist regelmäßig ein Abgaskondensator vorzusehen. Bei der Messvorrichtung nach der Erfindung ist in zweckmäßiger Weise ein aufklappbarer Kofferdeckel mit einem solchen Abgaskondensator versehen. In einer Offenstellung des Deckels, vertikal nach oben verriegelt, kann die Schwerkraft in ein Kondensationsverfahren einbezogen werden. Für eine Reinigung, einen Filteraustausch oder dergleichen wird der Abgaskondensator zweckmäßigerweise lösbar an dem Kofferdeckel festgelegt sein.

Eine frontseitige Klappe verbessert darüber hinaus noch die Zugänglichkeit des Kofferinneren.

Die Messvorrichtung nach der Erfindung wird anhand der Zeichnung näher erläutert, in der lediglich ein Ausführungsbeispiel schematisch dargestellt ist. In der Zeichnung zeigt:
- Fig. 1:: eine schematisierte Frontansicht der Messvorrichtung insgesamt und
- Fig. 2:: einen vergrößerten Schnitt durch das Wiegemodul mit angeschlossenem Anzeige- und Bedienmodul.

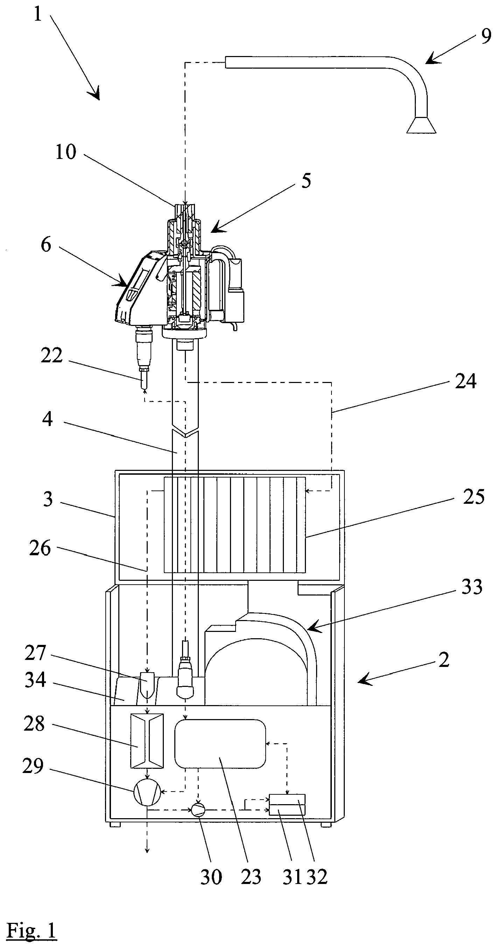

Figur 1 zeigt schematisch eine Messvorrichtung 1 nach der Erfindung, die vollständig für einen Transport oder zur Aufbewahrung in einem Koffer 2 unterbringbar ist. Der Koffer 2 weist einen aufklappbaren Kofferdeckel 3 auf, der, wie dargestellt, in einer vertikalen Stellung verriegelbar ist. Eine frontseitig, in Figur 1 nicht dargestellte Klappe, die sich etwa über die halbe Kofferhöhe erstreckt, verbessert die Zugänglichkeit des Kofferinneren.

Ist der Kofferdeckel 3 geöffnet, kann an einem teleskopierbaren Stativ 4 ein Wiegemodul 5 mit angeschlossenem Anzeige- und Bedienmodul 6 aus dem Kofferinneren auf eine bequeme Arbeitshöhe ausgezogen werden. Hierbei ist eine Handhabe 7 hilfreich, an der auch ein Hebel 8 bspw. einer Klemmvorrichtung angeordnet ist, mit der das Wiegemodul 5 mit dem Anzeige- und Bedienmodul 6 an dem Stativ 4 lösbar arretiert werden kann.

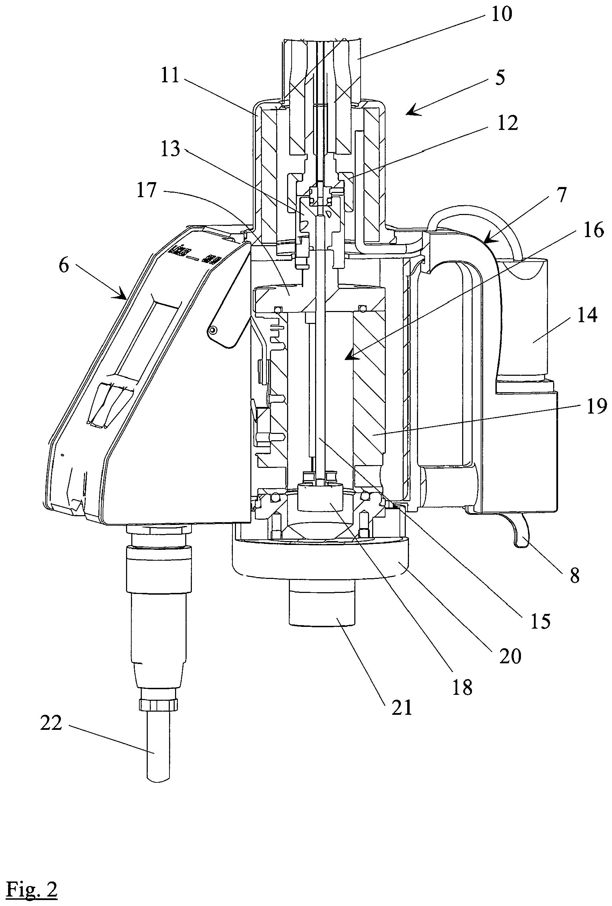

Von einer in einen Abgasstrom für eine Messung einzubringenden Messsonde 9 führt ein beheizbarer Ansaugschlauch 10 hin zu dem Wiegemodul 5 und ist dort lösbar angeschlossen. Nach Abziehen einer Schlauchkappe 11 und Lösen einer Überwurfmutter 12 kann der Ansaugschlauch von einem Schlauchtemperatursensor 13 abgezogen werden und kann die elektrische Verbindung zu dem Wiegemodul 5 nach Lösen eines Steckers 14 getrennt werden, vgl. Fig. 2, so dass ein einfaches Reinigen des Ansaugschlauchs 10, abgesetzt von der Messvorrichtung 1, möglich ist.

Andernends ist der Schlauchtemperatursensor 13 an einem Röhrchen 15 der Wägevorrichtung 16 angeschlossen.

Das Röhrchen 15 ist in einem Deckel 17 einerends fest eingespannt und andernends schwingfähig frei endend von einer aufgesteckten Filterpatrone 18 abgeschlossen.

Das Röhrchen 15 durchsetzt zentral eine evakuierbare Hülse 19, oberseitig von dem Deckel 17 und unterseitig von einem Verschlussdeckel 20 mit Schlauchanschlussstutzen 21 abgeschlossen.

In der Wandung der Hülse 19 sind, in der Zeichnung nicht dargestellt, Axialbohrungen eingebracht, die der Aufnahme von Heizpatronen dienen, durch die ein Aufheizen auch der Wägevorrichtung 16 möglich ist.

Von dem Anzeige- und Bedienmodul 6, in dem auch zweckmäßigerweise die Wiegeelektronik angeordnet ist, führt ein elektrisches Verbindungskabel 22 in das Kofferinnere und ist dort mit einer Steuerelektronik 23 verbunden.

Von dem Schlauchanschlussstutzen 21 des Wiegemoduls 5 führt ein in Fig. 1 lediglich angedeuteter Verbindungsschlauch 24 zu einem Abgaskondensator 25. Der Abgaskondensator 25 ist lösbar an dem vertikal arretierten Kofferdeckel 3 festgelegt. Ausgangseitig ist an dem Abgaskondensator 25 ein weiterer, lediglich angedeuteter Verbindungsschlauch 26 angeschlossen, der andernends über einen Schlauchanschluss 27 mit nicht dargestelltem Filter an eine symbolisch wiedergegebene Messvorrichtung 28 für ein Messen des Normvolumenstroms angeschlossen ist.

Den für den Gasdurchsatz notwendigen Differenzdruck erzeugt eine Pumpe 29, beispielsweise eine von der Steuerelektronik 23 geregelte Drehschieberpumpe, nach der das abgesaugte Gas in die Umgebung austritt.

Von einer weiteren Pumpe 30, beispielsweise einer von der Steuerelektronik 23 geregelte Membranpumpe, werden weitere Sensoren wie bspw. ein Sensor 31 für O₂ und/oder ein Sensor 32 für CO mit dem Abgas beaufschlagt, deren Messergebnisse gleichfalls von der Steuerelektronik 23 ausgewertet und von dem Anzeige- und Bedienmodul 6 angezeigt werden können.

Nach Beendigung einer Messung wird das Stativ 4 mit Wiegemodul 5 und Anzeige- und Bedienmodul 6 wieder in den Koffer 2 eingeschoben und wird der Ansaugschlauch 10 auf einer Haltevorrichtung 33 abgelegt. Daneben überdeckt eine Abdeckplatte 34 die Sensorik mit Sensoren 31,32, Messvorrichtung 28 und Steuerelektronik 23 sowie die Pumpen 29, 30.

## Patentansprüche

1. Messvorrichtung (1) für die Staubmessung im Abgas von Kleinfeuerungsanlagen für feste Brennstoffe, aufweisend
- eine Messsonde (9),
- eine Wägevorrichtung (16) mit einer Filtervorrichtung,
- einen die Messsonde (9) mit der Wägevorrichtung (16) verbindenden, beheizten Ansaugschlauch (10),
- ein Wiegemodul (5) mit einem angeschlossenen Anzeige- und Bedienmodul (6), wobei die Wägevorrichtung (16) in dem Wiegemodul (5) thermisch isoliert ausgebildet ist;
- einen Koffer (2);
- ein teleskopierbares Stativ (4), wobei das Wiegemodul (5) eigenständig ausgebildet und kombiniert mit dem Anzeige- und Bedienmodul (6) an dem teleskopierbaren Stativ (4) aus dem Koffer (2) ausziehbar ist.

2. Messvorrichtung nach Anspruch 1, **dadurch gekennzeichnet, dass** die Wägevorrichtung in einer beheizbaren Hülse (19) in dem Wiegemodul (5) angeordnet ist.

3. Messvorrichtung nach Anspruch 2, **dadurch gekennzeichnet, dass** ein Beheizen der Wägevorrichtung (16) impulsartig erfolgt.

4. Messvorrichtung nach einem oder mehreren der vorangehenden Ansprüche, **dadurch gekennzeichnet, dass** die Beheizung des Ansaugschlauches (10) geregelt erfolgt.

5. Messvorrichtung nach einem oder mehreren der vorangehenden Ansprüche, **dadurch gekennzeichnet, dass** ein Schlauchtemperatursensor (13) vorgesehen ist, angeordnet zwischen der Wägevorrichtung (5) und dem Ansaugschlauch (10) .

6. Messvorrichtung nach einem oder mehreren der Ansprüche 2 bis 5, **dadurch gekennzeichnet, dass** in der Hülse (19) ein schwingungsfähiges Röhrchen (15) angeordnet ist, das gasaustrittsseitig frei endend von einer aufgesteckten Filterpatrone (18) abgeschlossen ist.

7. Messvorrichtung nach einem oder mehreren der vorangehenden Ansprüche, **dadurch gekennzeichnet, dass** ein aufklappbarer Kofferdeckel (3) mit einem Abgaskondensator (25) versehen ist.

8. Messvorrichtung nach einem oder mehreren der vorangehenden Ansprüche, **dadurch gekennzeichnet, dass** der Koffer (2) eine frontseitige Klappe aufweist.

## Claims

1. A measuring device (1) for dust measurement in the exhaust gas of small furnaces for solid fuels, having
- a measuring probe (9),
- a weighing device (16) having a filter device,
- a heated suction hose (10) connecting the measuring probe (9) to the weighing device (16),
- a weighing module (5) with a connected display and operating module (6), wherein the weighing device (16) is constructed in the weighing module (5) in a thermally insulated manner;
- a case (2);
- a telescopable stand (4), wherein the weighing module (5) is of stand-alone construction and can be pulled out of the case (2) on the telescopable stand (4) combined with the display and operating module (6).

2. The measuring device according to Claim 1, **characterized in that** the weighing device is arranged in a heatable sleeve (19) in the weighing module (5).

3. The measuring device according to Claim 2, **characterized in that** heating of the weighing device (16) takes place in a pulsed manner.

4. The measuring device according to one or more of the preceding claims, **characterized in that** the heating of the suction hose (10) takes place in a regulated manner.

5. The measuring device according to one or more of the preceding claims, **characterized in that** a hose temperature sensor (13) is provided, arranged between the weighing device (5) and the suction hose (10).

6. The measuring device according to one or more of Claims 2 to 5, **characterized in that** a small vibratory tube (15) is arranged in the sleeve (19), which is closed in a freely ending manner on the gas outlet side by a filter cartridge (18), which is plugged on.

7. The measuring device according to one or more of the preceding claims, **characterized in that** a case lid (3), which can be flipped open, is provided with an exhaust gas condenser (25).

8. The measuring device according to one or more of the preceding claims, **characterized in that** the case (2) has a front-side flap.

## Revendications

1. Dispositif de mesure (1) pour la mesure des poussières dans les gaz d'échappement des petites installations de combustion pour des combustibles solides, présentant :
- une sonde de mesure (9),
- un dispositif de pesée (16) comprenant un dispositif de filtre,
- un flexible d'aspiration (10) chauffé reliant la sonde de mesure (9) au dispositif de pesée (16),
- un module de balance (5) avec un module d'affichage et d'utilisation (6) relié à celui-ci, dans lequel le dispositif de pesée (16) est conçu isolé thermiquement dans le module de balance (5) ;
- un coffret (2) ;
- un pied télescopique (4), dans lequel le module de balance (5) est conçu autonome et peut être déployé hors du coffret (2), sur le pied télescopique (4), combiné au module d'affichage et d'utilisation (6).

2. Dispositif de mesure selon la revendication 1, **caractérisé en ce que** le dispositif de pesée est disposé dans un manchon pouvant être chauffé (19) dans le module de balance (5).

3. Dispositif de mesure selon la revendication 2, **caractérisé en ce qu'**un chauffage du dispositif de pesée (16) s'effectue par impulsions.

4. Dispositif de mesure selon une ou plusieurs des revendications précédentes, **caractérisé en ce que** le chauffage du tube d'aspiration (10) s'effectue de manière régulée.

5. Dispositif de mesure selon une ou plusieurs des revendications précédentes, **caractérisé en ce qu'**un capteur de température de flexible (13) est prévu, disposé entre le dispositif de pesée (5) et le flexible d'aspiration (10).

6. Dispositif de mesure selon une ou plusieurs des revendications 2 à 5, **caractérisé en ce qu'**un petit tuyau (15) pouvant osciller est disposé dans le manchon (19), qui est fermé par une cartouche de filtre (18) et débouchant librement côté sortie des gaz.

7. Dispositif de mesure selon une ou plusieurs des revendications précédentes, **caractérisé en ce qu'**un couvercle de coffret (3) rabattable avec un condensateur de gaz d'échappement (25) est prévu.

8. Dispositif de mesure selon une ou plusieurs des revendications précédentes, **caractérisé en ce que** le coffret (2) présente un volet avant.
